# EUROPÄISCHE PATENTSCHRIFT

(11) **EP 3 827 425 B1**
(45) Veröffentlichungstag und Bekanntmachung des Hinweises auf die Patenterteilung: **07.09.2022**
(21) Anmeldenummer: 19752640.3
(22) Anmeldetag: 24.07.2019
(51) Int. Cl.: G09B 21/00, A61F 11/04

(54) **VERFAHREN UND VORRICHTUNG ZUR STEIGERUNG DER MUSIKEMOTIONALITÄT**
METHOD AND DEVICE FOR INCREASING MUSICAL SENSITIVITY
PROCÉDÉ ET DISPOSITIF POUR L'ACCROISSEMENT DE L'ÉMOTIVITÉ MUSICALE

(30) Priorität: 24.07.2018 DE 102018006210
(43) Veröffentlichungstag der Anmeldung: 02.06.2021
(73) Patentinhaber: Feelbelt GmbH, 14482 Potsdam (DE)
(72) Erfinder: HANSEN, Jens, 12109 Berlin (DE)
(74) Vertreter: Bittner, Thomas L.
(86) Internationale Anmeldenummer: PCT/DE2019/100675
(87) Internationale Veröffentlichungsnummer: WO 2020/020414

(56) Entgegenhaltungen:
- WO-A1-2010/020201
- WO-A2-2006/092136
- CN-U- 203 122 761
- DE-A1-102007 012 315
- US-A1- 2007 242 040
- US-A1- 2011 129 093
- US-A1- 2017 353 778
- MARIA KARAM ET AL: "The emoti-chair", PROCEEDINGS OF THE 28TH OF THE INTERNATIONAL CONFERENCE EXTENDED ABSTRACTS ON HUMAN FACTORS IN COMPUTING SYSTEMS, CHI EA '10, ACM PRESS, NEW YORK, NEW YORK, USA, 10. April 2010 (2010-04-10), Seiten 3069-3074, XP058182575, DOI: 10.1145/1753846.1753919 ISBN: 978-1-60558-930-5
- KARAM M ET AL: "Designing the Model Human Cochlea: An Ambient Crossmodal Audio-Tactile Display", IEEE TRANSACTIONS ON HAPTICS, IEEE, USA, Bd. 2, Nr. 3, 1. Juli 2009 (2009-07-01), Seiten 160-169, XP011327940, ISSN: 1939-1412, DOI: 10.1109/TOH.2009.32

## Beschreibung

Die Erfindung betrifft ein Verfahren und einer Vorrichtung zur Steigerung der Wahrnehmung von akustischen Ereignissen oder Signalen, insbesondere von Musik, indem aus dieser vorzugsweise Vibrationen abgeleitet und auf die Haut übertragen werden.

Aus der WO 2006 / 092136 A2 sind ein Verfahren und eine Vorrichtung zum sensitiven Erfassen von Schallereignissen zur Erzielung von Gefühls- oder Empfindungszuständen der lebenden Haut bekannt. Dabei wird nur der fühlbare Frequenzbereich der Schallereignisse in Vibrationen umgesetzt, indem das Schallereignis spektral zerlegt und im Wesentlichen der momentan dominante Spektralbereich zur Ansteuerung der Vibrationsgeber verwendet wird, wobei die Anzahl der Vibrationsgeber von der Frequenzlage dieser Spektralbereiche abhängig ist. Dabei wird nicht offenbart, wie diese Ableitung vorgenommen wird, um eine Korrelation von gehörtem und gefühltem Schallereignis, insbesondere von Musik, zu erzielen. Dem Schaltschema für die Vibrationsgeber werden weiterhin die Amplituden der Spektren zugrunde gelegt. Diese sind jedoch nicht maßgeblich für die Vibrationsstärke der Vibrationsgeber.

Darauf aufbauend sollte mit der WO 2010 / 020 201 A1 erreicht werden, dass auch Musikinhalte oberhalb des fühlbaren Frequenzbereiches, also etwa oberhalb von 600 Hertz, erfasst werden. Mit der dargestellten Realisierung kann dies jedoch nicht erreicht werden, da die Amplitude eines Spektrums nicht der Amplitude des zeitlichen Signals mit diesem Spektrum entspricht.

In Verfahren, bei denen Schallereignisse für Gehörgeschädigte in erlernte sensorische Wahrnehmungsmuster umgesetzt werden, werden inhaltlich definierte akustische Signale sensorisch an gehörlose Personen übermittelt, sodass diese und von diesen verstanden werden können. Solche Lösungen beruhen auf einem vorangegangenen Lernprozess und sind beispielsweise bekannt aus US 5,035,242, US 4,167,189, EP 0 766 218 A1, US 4,250,637, US 4,390,756 oder DE 39 24 708 A1.

Es wurden ein Modell Human Cochlea (MHC), eine sensorische Substitutionstechnik und ein System vorgeschlagen (Karam et al., "Designing the Model Human Cochlea: An Ambient Crossmodal Audio-Tactile Display", IEEE, Bd. 2, Nr. 3, 1. Juli 2009, Seiten 160-169), bei denen auditive Informationen in vibrotaktile Stimuli gewandelt werden und hierbei ein taktiles Display verwendet wird. Das Modell wird verwendet, um Musik in diskrete Vibrationssignale zu wandeln, die entlang des Rückens des Körpers mit Hilfe eines Stuhlformfaktors angezeigt werden. Schwingungsspulen erleichtern die direkte Übersetzung der auditiven Informationen in die mehreren diskreten vibrotaktilen Kanäle, was das Potenzial erhöht, Abschnitte der Musik zu identifizieren, die andernfalls durch das kombinierte Signal überdeckt würden. Ein Ziel war es, die Zugänglichkeit zu den emotionalen Informationen, die in der Musik zum Ausdruck kommen, für gehörlose oder schwerhörige Nutzer zu verbessern.

Aufgabe der Erfindung ist es, die Wahrnehmung von akustischen Ereignissen, insbesondere die Musikemotionalität zu steigern. Insbesondere sollen die einwirkenden Vibrationen auf die Haut wie auch die Wahl der Einwirkungsorte, bei welchen eine hohe Sensitivität für äußere Einflüsse vorliegen sollte, optimal hinsichtlich der Korrelation zur Musik und des Differenzierungs- und Wahrnehmungsvermögens der Haut angepasst werden.

Eine Korrelation von gehörten und gefühlten Wahrnehmungen ist wichtig, da beide Eindrücke sonst als nicht zusammengehörig, d.h. als Fremdeindrücke gewertet würden und eine Steigerung der Wahrnehmung, z.B. Intensivierung des Musikerlebnisses somit nicht gegeben wäre.

Insbesondere soll die zweite Wahrnehmung, also die taktile Wahrnehmung durch Vibrationen, direkt auf den Hörer einwirken, ihn also gleichsam anfassen und sich in ihr die gehörte Wahrnehmung gleichsam widerspiegeln, um eine Steigerung der Wahrnehmung, insbesondere der Emotionalität zu bewirken.

Gelöst wird die Aufgabe durch ein Verfahren und eine Vorrichtung mit den Merkmalen der unabhängigen Ansprüche, Vorteilhafte Ausgestaltungen sind Gegenstand der Unteransprüche.

Zum besseren Verständnis werden einige der verwendeten Begriffe vorab erläutert.
- Basisspektrum ist ein aus dem Frequenzspektrum eines akustischen Ereignisses bestimmtes Frequenzspektrum, insbesondere ein ausgewählter Teil des gesamten Frequenzspektrums. Es kann sich um ein mittleres ausgewähltes Frequenzspektrum handeln, das aufgrund des aktuell vorliegenden Frequenzspektrums des akustischen Ereignisses, z.B. einer aktuell gehörten Musik, angepasst wird, indem es schmaler oder breiter ausgebildet wird. Insbesondere wird als Basisspektrum somit derjenige Spektralbereich bezeichnet, ein welchem die wesentlichen Inhalte eines akustischen Signals liegen.
- Teilspektren sind Spektralbereiche, in welche sich das Basisspektrum aufteilen lässt. Insbesondere ist das Basisspektrum in Teilspektren unterteilt, vorzugsweise derart, dass die Teilspektren in Summe das gesamte Basisspektrum ergeben. Die Anzahl der Teilspektren kann vorgegeben sein, insbesondere der Anzahl von Vibrationsgebern entsprechen. Die Teilspektren können in ihrer Breite gleich oder unterschiedlich sein.
- Mit Amplitude wird der mittlere Pegel des zeitlichen Verlaufs eines Spektralbereichs bezeichnet.
- Steuerspannungen sind die Spannungen zum Ansteuern der Vibrationsgeber und werden aus dem Frequenzbereich zumindest eines der Teilspektren abgeleitet.
- Akustische Ereignisse umfassen jede Art von Schallereignissen, deren Wahrnehmung während des Ereignisses durch die Erzeugung von Vibrationen gesteigert werden soll, wobei das Ziel der Wahrnehmungssteigerung im Wesentlichen in der Erhöhung der mit einem Musikstück beabsichtigten emotionalen Wirkung besteht. Überdies kann auch eine Steigerung der Musikerkennbarkeit bewirkt werden, etwa bei starken Nebengeräuschen oder leiser Musik. Akustische Ereignisse umfassen auch Stimmen, Klänge, Geräusche oder Töne (z.B. Ton zu Bild- / Videomaterial, wie Ton zu einem Film, Töne eines Videospiels, usw.), falls der Begriff "Musik" verwendet wird.

Es ist ein Verfahren zur Steigerung der Wahrnehmung von akustischen Ereignissen, insbesondere zur Steigerung der Musikemotionalität bereitgestellt. Dabei werden aus einem akustischen Ereignis abgeleitete und mit dem akustischen Ereignis korrelierende Vibrationen mittels Vibrationsgebern auf die Haut einer Person übertragen, wobei die Vibrationsgeber an Positionen in einer vorgegebenen Anordnung auf der Haut räumlich verteilt sind. Mittels einer Steuerung, welcher ein das akustische Ereignis wiedergebende Eingangssignal zugeführt wird, werden Steuerspannungen bestimmt und die Vibrationsgeber jeweils mit einer der ermittelten Steuerspannungen zur Erzeugung einer auf der Haut fühlbaren Vibration angesteuert.

Aus dem Frequenzspektrum des akustischen Ereignisses wird ein Basisspektrum bestimmt, insbesondere als Teil des Frequenzspektrums ausgewählt, wobei das Basisspektrum in Teilspektren unterteilt wird, welche jeweils einen Frequenzbereich des Basisspektrums umfassen. Die Steuerspannungen werden dann jeweils aus dem Frequenzbereich eines zugeordneten Teilspektrums (oder gegebenenfalls mehrerer) abgeleitet. Zwischen Frequenzlagen der Steuerspannungen und den Positionen der Vibrationsgeber und/oder zwischen den Teilspektren und den Positionen der Vibrationsgeber besteht dabei eine Zuordnung.

Durch geeignete Bestimmung des Basisspektrums aus dem gesamten Frequenzspektrum eines akustischen Ereignisses wird die Grundlage für eine Steigerung der Wahrnehmung geschaffen. Die Auswahl des Basisspektrums kann dabei insbesondere von der Art des akustischen Ereignisses abhängen. Beispielsweise kann das Basisspektrum bei basslastiger Musik in einem Bereich niedrigerer Frequenzen gewählt werden als bei höhenlastiger Musik. Auch kann durch geeignete Wahl der Breite des Basisspektrums die Wahrnehmung gesteigert werden. Beispielsweise kann auf diese Weise auch bei akustischen Ereignissen, bei welchen der Hauptteil der Frequenzen in einem schmalen Bereich liegt, durch entsprechend gewählte Breite des Basisspektrums eine Verteilung auf alle Vibrationsgeber erreicht werden. Ohne Auswahl eines solchen Basisspektrums würden beispielsweise bei basslastiger Musik diejenigen Vibrationsgeber, welche Teilspektren mit hohen Frequenzen zugeordnet sind, kaum zur Vibration angeregt. Neben der Auswahl des Basisspektrums wird die Wahrnehmung weiter durch eine Zuordnung der Vibrationsgeber, insbesondere bezüglich deren Position innerhalb der Anordnung, gesteigert, sodass Töne unterschiedlicher Frequenzen an unterschiedlichen Stellen auf der Haut wahrgenommen werden können.

Während das Basisspektrum fest vorgegeben sein oder beispielsweise nur beim Start einmalig bestimmt werden kann, ist es vorteilhaft, wenn das Basisspektrum variabel ist, insbesondere an das aktuelle akustische Ereignis laufend oder zumindest in vorgegebenen zeitlichen Abständen während des aktuellen akustischen Ereignisses angepasst wird. Durch diese "dynamische" Anpassung des Basisspektrums kann zu jeder Zeit während des akustischen Ereignisses eine gesteigerte Wahrnehmung erreicht werden.

Insbesondere kann das Bestimmen des Basisspektrums ein Erweitern und/oder Reduzieren des Basisspektrums in der Breite unter Beibehaltung der Anzahl der Teilspektren umfassen, wobei das Basisspektrum in der Breite reduziert wird, wenn in einer Zeiteinheit eine Anzahl von Spektralkomponenten im Frequenzbereich des akustischen Ereignisses in den Randbereichen des aktuellen Basisspektrums (d.h. innerhalb eines vorgegebenen Abstands vom jeweiligen Rand) auftritt, die unterhalb eines vorgegebenen Schwellenwerts liegt, insbesondere keine Spektralkomponente, und wobei das Basisspektrum in der Breite erweitert wird, wenn eine Spektralkomponente im Frequenzspektrum des akustischen Ereignisses auftritt, die außerhalb des ausgewählten Basisspektrums liegt.

Weiterhin ist eine Vorrichtung zur Steigerung der Wahrnehmung akustischer Ereignisse bereitgestellt, welche eingerichtet ist, das hier beschriebene Verfahren auszuführen. Die Vorrichtung weist eine Mehrzahl von Vibrationsgebern zum Übertragen von Vibrationen auf die Haut einer Person sowie eine mit den Vibrationsgebern verbundene Steuerung zur Ansteuerung der Vibrationsgeber mit einer Steuerspannung zur Erzeugung der Vibrationen auf. Die Vibrationsgeber sind an Positionen in einer vorgegebenen Anordnung derart angeordnet, dass sie auf der Haut der Person räumlich verteilbar sind, wobei aus einem akustischen Ereignis abgeleitete und mit dem akustischen Ereignis korrelierende Vibrationen mittels den Vibrationsgebern auf die Haut einer Person zu übertragbar sind. Die Steuerung ist eingerichtet, ein zu dem akustischen Ereignis gehörendes Eingangssignal zu empfangen, Steuerspannungen zu bestimmen und die Vibrationsgeber jeweils mit einer der ermittelten Steuerspannungen zur Erzeugung einer auf der Haut fühlbaren Vibration anzusteuern.

Die Vorrichtung kann beispielsweise als ein um die Hüfte oder Taille zu tragender Gürtel, als Band oder als Kleidungsstück (z.B. als Weste oder T-Shirt) ausgebildet sein, vorzugsweise derart, dass bei Benutzung die Vibrationsgeber auf der Haut im Bereich des Bauchs und der Taille oder Hüfte der Person platzierbar sind, was insbesondere aufgrund der Nähe zum vegetativen Nervensystem vorteilhaft ist. insbesondere können die Vibrationsgeber derart angeordnet sein, dass sie beim Tragen der Vorrichtung durch die Person mit der Haut der Person in Kontakt kommen oder zumindest derart relativ zur Haut der Person, dass eine Vibration auf die Haut übertragbar ist. Beispielsweise können die Vibrationsgeber zur Erhöhung des Tragekomforts in die Vorrichtung integriert, beispielsweise von einer Stoffschicht bedeckt sein.

Gemäß einem weiteren Aspekt ist ein Verfahren zur Steigerung der Musikemotsonalität bereitgestellt, indem aus der hörbaren Musik abgeleitete mit der hörbaren Musik korrelierende Vibrationen von Vibrationsgebern auf die Haut übertragen werden, wobei die Vibrationsgeber räumlich verteilt in einen um die Hüfte oder Taille zu tragenden Gürtel angeordnet sind und von in unterschiedliche Frequenzbereiche gefilterten Musiksignalen al Steuerspannung angesteuert werden, sieht vor, dass
ausgehend von einer mittleren spektralen Breite eines Basisspektrums aus dem Musikspektrum, wobei das Basisspektrum in Teilspektren unterteilt ist,
das Basisspektrum in der Breite erweitert wird unter Beibehaltung der Anzahl der Teilspektren, sobald eine Spektralkomponente der aktuellen Musik auftritt, die außerhalb des aktuellen Basisspektrums liegt und/oder
das Basisspektrum in der Breite reduziert wird unter Beibehaltung der Anzahl der Teilspektren, wenn in einer Zeiteinheit keine Spektralkomponente der aktuellen Musik in den äußeren Bereichen des aktuellen Basisspektrums auftritt.
wobei mit den zeitlichen Verläufen der Teilspektren die Vibrationsgeber angesteuert werden und wobei zwischen der Frequenzlage der Steuerspannungen und der Position der Vibrationsgeber ein rechentechnisch abrufbares Zuordnungsschema besteht.

Gemäß einer bevorzugten Ausführung der oben beschriebenen Verfahren ist die Anzahl der von einer Steuerspannung angesteuerten Vibrationsgeber von dem Amplitudenverhältnis der einzelnen Steuerspannungen abhängig. wobei bei gleicher oder im Wesentlichen gleicher Amplitude (d.h. bis zu einer vorgegebenen Abweichung) der Steuerspannungen aller Teilspektren die Steuerspannung den Vibrationsgebern in der Reihenfolge ihrer Frequenzlage zugeführt wird, und bei Ungleichheit bzw. ab einer vorgegebenen Abweichung der Amplitude der Steuerspannungen der Teilspektren die dominierende Steuerspannung für den Vibrationsgeber dieses Spektrums sowie für die Vibrationsgeber von Teilspektren mit einer höheren Frequenzlage vorgesehen ist.

Es kann vorgesehen sein, dass mit der Steuerspannung, welche in der Frequenzlage unterhalb der Steuerspannung mit dem maximalen Wert die höchste Amplitude aufweist auch ein oder mehrere Vibrationsgeber, welche ansonsten den zwischen diesen beiden Steuerspannungen liegenden Frequenzbereichen zugeordnet wären, angesteuert werden, und dass eine sinngemäße Zuordnung für die Steuerspannungen unterhalb des angesprochenen Frequenzbereichs besteht.

Die Einwirkurigsbreiten der gefilterten Eingangssignale im fühlbaren Spektralbereich werden also je nach Frequenzlage und Amplitudendominanz erweitert.

Unabhängig von dieser Zuordnung der Steuerspannungen ist es für das Fühlbarmachen der gehörten Musik vorteilhaft, wenn mindestens ein Vibrationsgeber permanent mit der höchstfrequenten Steuerspannung der Teilspektren angesteuert wird.

Bevorzugt sind die Vibrationsgeber symmetrisch und paarweise bezogen auf eine Mittellinie der Anordnung der Vibrationsgeber angeordnet, insbesondere bezüglich einer Verteilungsrichtung, in welcher sich die Anordnung erstreckt, und werden paarweise oder einzeln angesteuert. Die Mittellinie der Anordnung kann dabei der Mittellinie der Vorrichtung, beispielsweise der Mittellinie eines Gürtels entsprechen. Die Vibrationsgeber können dabei insbesondere entlang einer Linie angeordnet sein. Es ist jedoch auch denkbar, dass die Vibrationsgeber beabstandet in der Verteilungsrichtung sind, jedoch nicht auf einer Reihe oder Linie liegen. Es können gleichermaßen auch mehr als eine Reihe von Vibrationsgebern nebeneinander vorgesehen sein, mit der gleichen Anzahl oder unterschiedlichen Anzahl von Vibrationsgebern pro Reihe. Die Vibrationsgeber können auch beliebig angeordnet sein, solange eine Zuordnung wie oben beschrieben besteht. Vorzugsweise erhöht sich die Frequenz der Teilspektren in Richtung Mittellinie z.B. des Gürtels. Mit anderen Worten, Vibrationsgeber, die näher an der Mittellinie sind, werden Teilspektren in einem höheren Frequenzbereich zugeordnet, und werden vorzugsweise mit einer höherfrequenten Steuerspannung zu einer höherfrequenten Vibration angeregt, als Vibrationsgeber, die weiter von der Mittellinie entfernt sind. Eine solche Ansteuerung, bei weicher also insbesondere die Höhen am Bauch und die Tiefen (Bässe) an den Seiten durch entsprechende Vibrationen fühlbar gemacht werden, hat sich aufgrund der unterschiedlichen Sensitivitäten der Haut an verschiedenen Stellen als vorteilhaft für die Steigerung der Wahrnehmung eines akustischen Ereignisses, insbesondere die Steigerung der Musikernotionalität herausgestellt. Durch die Nähe der sensitiven Einwirkungen im Taillenbereich zum vegetativen Nervensystem werden diese besonders sensibel wahrgenommen.

Bei dem Vibrationsgeber, der permanent mit der höchstfrequenten Steuerspannung der Teilspektren angesteuert wird, handelt es sich bei der paarweisen Anordnung bevorzugt um das unmittelbar rechts und links der Mittelinie befindliche Vibrationsgeberpaar.

Das Verfahren schließt auch Verfahrensschritte ein, mit welchen Musikphasen im nichtfühlbaren Frequenzbereich, weiche deutlich die Musik prägen, fühlbar gemacht werden. Mit anderen Worten, es kann Bereiche im Frequenzspektrums eines akustischen Ereignisses geben, welche bezüglich ihrer Frequenz zwar hörbar sind (oder zumindest zum Höreindruck beitragen), aber mit dieser Frequenz auf der Haut nicht fühlbar wären.

So kann vorgesehen sein, dass die im höchsten noch fühlbaren Teilspektrum liegende Steuerspannung in der Amplitude angehoben wird, mit anderen Worten, dass bei Auftreten von Signalanteilen des Eingangssignals in einem nicht fühlbaren Teilspektrum die im höchsten noch fühlbaren Teilspektrum liegende Steuerspannung in der Amplitude angehoben wird.

Weiter kann ab einer bestimmten Dominanz der Musikphase im nichtfühlbaren Frequenzbereich, die im höchsten noch fühlbaren Teilspektrum liegende Steuerspannung mehreren Vibrationsgebern als Steuerspannung zugeführt werden. Hierdurch würde sich die Einwirkungsbreite dieser Musikphase erhöhen.

Die Erfindung sieht vor, dass der Amplitudenverlauf des im nicht fühlbaren Frequenzbereich liegenden Eingangssignals einer im fühlbaren Bereich Frequenzbereich liegenden Oszillationsspannung als Amplitudenverlauf aufgeprägt und diese als Steuerspannung den Vibrationsgebern zugeführt wird.

Die Amplitudenverlaufsübernahme sieht vor, dass der zur Trennung des nicht fühlbaren vom fühlbaren Frequenzbereich zum Einsatz kommende Selektionsverstärker eine dynamische Arbeitspunkteinstellung aufweist, derart, dass das gleichgerichtete Ausgangssignal des Verstärkers gegenphasig über eine zeitliche Verzögerungsstufe auf den Verstärkereingang rückgekoppelt wird, wodurch eine von der Änderungssteilheit des im nichtfühlbaren Frequenzbereich liegenden Musiksignals abhängige Verstärkung erreicht wird, und dass der Amplitudenverlauf des auf diese Weise erzeugten Ausgangssignals des Verstärkers der im fühlbaren Bereich liegenden Oszillatorspannung als Amplitudenverlauf aufgeprägt wird, welche dann als Steuerspannung für die Vibrationsgeber verwendet wird.

Die zeitliche Verzögerung des rückgekoppelten Signals ist so bemessen, dass diese relativ schnellen Anstiegen des Musiksignals nicht folgt, also bei derartigen Musikphasen die vollständige Verstärkung wirksam ist, bei Verharren des Musiksignals jedoch auf dem höheren Niveau die Verstärkung gemäß der Zeitkonstante des rückgekoppelten Signals aufgrund seiner Gegenphasigkeit herabgesetzt und der Arbeitspunkt in den unteren Spannungsbereich verlagert wird. Auf einen erneuten schnellen Anstieg wirkt dann wieder die Grundverstärkung, wobei sich das verstärkte Signal aufgrund der niedrigen Ausgangsspannung vollständig in den oberen Spannungsbereich ausprägen kann.

Bei konstanter Arbeitspunktlage wären Signalschwankungen ab einer gewissen Basissignalamplitude nicht erkennbar, da diese außerhalb des Arbeitsbereichs - also im Sättigungsbereich - des Verstärkers lägen. Anderseits wären bei reduzierter Verstärkung die Steuerspannungen für die Vibrationsgeber zu gering, um Signalschwankungen sensitiv erfassen zu können.

Durch die variable Arbeitspunktlage kann die Basisverstärkung etwa auf den fünffachen Wert erhöht werden, wodurch auch geringe Amplitudenschwankungen relativ hoher Signale sensitiv erfasst werden.

In einer weiteren Ausgestaltung kann vorgesehen sein, dass bei Signalanteilen der Musik in einem nichtführbaren Teilspektrum dieser Frequenzbereich über eine oder mehrere, vorzugsweise mindestens zwei Filterstufen in seiner Gesamtheit erfasst wird. Hierdurch wird der Tatsache Rechnung getragen, dass bei Musik - im Gegensatz zur Sprache - mehrere Signalquellen den Schall erzeugen. Durch die Erfassung des Frequenzbereichs über lediglich eine Filterstufe können sich die Modulationsinhalte der Einzelquellen, zum Beispiel durch interferenzbildung, gegenseitig verfälschen. Bei Verwendung mehrerer Filter nähert sich die Schaltung dem Selektionsvermögen des Gehörs an, wodurch eine stärkere Korrelation zwischen gehörten und gefühlten Wahrnehmungen erreicht wird.

Dabei kann das Zuordnungsschema der Steuerspannungen zu den Vibrationsgebern bei mindestens zwei Filterstufen derart vorgenommen werden, dass mit den aus den höherfrequenten Signal abgeleiteten Steuerspannungen die mehr zur Mittellinie hin befindlichen Vibrationsgeber angesteuert werden und mit den aus dem niederfrequenten Signal abgeleiteten Steuerspannungen, die von der Mittelinie weiter entfernten Vibrationsgeber.

Bei einer Amplitudendominanz einer Steuerspannung hat es sich dabei als vorteilhaft erwiesen, wenn weitere (oder zumindest ein weiterer) Vibrationsgeber mit dieser Spannung angesteuert werden, insbesondere Vibrationsgeber, welche zu dem eigentlich zugeordneten Vibrationsgeber benachbart sind.

Bei Musikstücken mit eingeschränktem Spektralbereich kann vorgesehen sein, dass die Aufteilung der Frequenzbereiche für die Steuerspannungen entsprechend der Gesamtbreite der Musik gespreizt wird. Das bedeutet, dass die Breite der Spektralbereiche, derer, Breite um eine Oktave betragen sollte, kleiner wird.

Eine weitere vorteilhafte Ausgestaltung sieht vor, dass über eine Benutzerschnittstelle, vorzugsweise über eine Software, wie einer App Steuerbefehle, insbesondere an die digitale Schaltung zur Umsetzung des Schaltschemas, und/oder zur Änderung der Zuordnung oder Schaltmatrix für die Vibrationsgeber übermittelbar sind,

Wie oben beschrieben ist eine entsprechende Vorrichtung, wie ein Gürtel mit den Vibrationsgebern vorgesehen, um das oben beschriebene Verfahren umzusetzen. Gemmäß einem weiteren Aspekt kann eine Vorrichtung zur Steigerung der Müsikemotionalität vorgesehen sein, indem aus der hörbaren Musik abgeleitete mit der hörbaren Musik korrelierende Vibrationen von Vibrationsgebern auf die Haut übertragen werden, wobei die Vibrationsgeber in einen um die Hüfte zu tragenden Gürtel angeordnet sind und von in unterschiedliche Teilspektren gefilterten Musiksignalen in Form einer Steuerspannung angesteuert werden sieht eine Elektronik aus schaltungstechnisch miteinander gekoppelte Komponenten vor nämlich eine Musikaufnahmekomponente, eine Schaltung zur Ableitung des Basisspektrums, steuerbare Filterschaltungen mit einer Schaltung zur Anpassung der gefilterten Signale, Schaltungen zur Amplitudenerkennung, Komparatorschaltungen, eine digitale Schaltung zur Umsetzung des Schaltschemas, steuerbare Verstärker, eine Schaltmatrix sowie die Vibrationsgeber,

Es versteht sich, dass statt der beschriebenen analogen Schaltung vorteilhaft eine digitale Schaltung (z.B. Microcontroller) und/oder Software als Steuerung verwendet werden kann. Diese empfängt ein digitales Eingangssignal, führt die beschriebenen Schritte zur Ermittlung der Steuerspannungen aus und veranlasst die Ansteuerung der Vibrationsgeber mit der jeweiligen Steuerspannung.

Die Steuerung bzw Elektronik ist vorzugsweise in die Vorrichtung integriert und verfügt über mindestens eine Schnittstelle zum Empfang des Eingangssignals, beispielsweise eine Schnittstelle zu einem Signalgeber in/oder an der Musikaufnahmekomponente. Bevorzugt kann vorgesehen sein, dass die Schnittstelle drahtlos ist, beispielsweise Bluetooth, welche das Eingangssignal von einem Ausgabegerät empfängt. Es versteht sich, dass die Schnittstelle auch kabelgebunden sein kann. Es kann weiterhin vorgesehen sein, dass das Eingangssignal über eine Schnittstelle ausgegeben bzw. weitergegeben wird, insbesondere an ein Gerät zum Abspielen, wie Kopfhörer oder Lautsprecher. Für eine Steigerung der Wahrnehmung soll durch die Übertragungen der Signale kein zeitlicher Versatz zwischen dem gehörten akustischen Ereignis und dessen Umsetzung in Vibrationen entstehen.

Weiterhin verfügt die Vorrichtung vorteilhaft über einen Energiespeicher, wie einen Akku, der mit der Steuerung gekoppelt ist, um diese mit elektrischer Energie zu versorgen. Das hat den Vorteil, dass die Vorrichtung autark nutzbar ist, wenn auch die Musiksignale funktechnisch übertagen werden. Der Nutzer ist so ortsunabhängig.

Die Vibrationsgeber sind bevorzugt innenseitig an der Vorrichtung angeordnet, sodass sie wie oben beschrieben die Vibrationen auf die Haut übertragen können, beispielsweise innenseitig am oder in dem Gürtel angeordnet.

Durch die Schaltmatrix können im Zusammenwirken mit der digitalen Schaltung zur Umsetzung des Schaltschemas mindestens folgende Ansteuerungen der Vibrationsgeber in Kombination oder einzeln vornehmbar sein, nämlich
- dass bei gleicher Amplitude der Steuerspannung aller Teilspektren die Steuerspannung den Vibrationsgebern in der Reihenfolge ihrer Frequenzlage zugeführt wird,
- dass bei Ungleichheit der Amplitude der Steuerspannungen der Teilspektren die dominierende Steuerspannung für den Vibrationsgeber dieses Teilspektrums sowie für ein oder mehrere Vibrationsgeber der Teilspektren mit einer höheren Frequenzlage vorgesehen ist,
- dass mindestens ein Vibrationsgeber permanent mit der höchstfrequenten Steuerspannung der Teilspektren angesteuert wird,
- dass mit der Steuerspannung, welche in der Frequenzlage unterhalb der Steuerspannung mit dem maximalen Wert die höchste Amplitude aufweist, auch ein oder mehrere Vibrationsgeber, welche zwischen diesen beiden Steuerspannungen liegen, angesteuert werden und dass eine sinngemäße Zuordnung für die Steuerspannungen in den darunter liegenden Frequenzbereichen besteht,
- dass bei die Musik prägenden Signalanteilen im nichtfühlbaren Frequenzbereich die Amplitude der im höchsten noch fühlbaren Frequenzbereich liegenden Steuerspannung angehoben und ab einer bestimmten Höhe der Signalanteile mehreren Vibrationsgebern zugeführt wird,
- dass bei Signalanteilen der Musik in einem nichtführbaren Teilspektrum dieser Frequenzbereich über mindestens zwei Filterstufen in seiner Gesamtheit erfasst wird,
- dass bei Signalanteilen der Musik in einem nicht fühlbaren Teilspektrum das Zuordnungsschema der Steuerspannungen zu den Vibrationsgebern bei mindestens zwei Filterstufen derart vorgenommen wird, dass mit den aus den höherfrequenten Signal abgeleiteten Steuerspannungen die mehr zur Mittellinie des Gürtels hin befindlichen Vibrationsgeber angesteuert werden und mit den aus dem niederfrequenten Signal abgeleiteten Steuerspannungen, die von der Mittelinie weiter entfernten Vibrationsgeber, und/oder
- das bei einer Amplitudendominanz einer Steuerspannung weitere Vibrationsgeber mit dieser Spannung angesteuert werden.

Der Amplitudenverlauf des im nicht fühlbaren Frequenzbereich liegenden Eingangssignals wird einer im fühlbaren Bereich Frequenzbereich liegenden Oszillationsspannung als Amplitudenverlauf aufgebprägt und diese als Steuerspannung den Vibrationsgebern zugeführt.

Der Selektionsverstärker weist zur Trennung des nichtfühlbaren vom fühlbaren Frequenzbereich eine dynamische Arbeitspunkteinstellung auf, derart, dass das gleichgerichtete Ausgangssignal des Verstärkers gegenphasig über eine zeitliche Verzögerungsstufe auf den Verstärkereingang rückgekoppelt wird, wodurch eine von der Änderungssteilheit des im nichtfühlbaren Frequenzbereich liegenden Musiksignals abhängige Verstärkung erreicht wird, und dass auf diese Weise erzeugte Ausgangssignal des Verstärkers der im fühlbaren Bereich liegenden Oszillatorspannung als Amplitudenverlauf aufgeprägt wird, welche dann als Steuerspannung für die Vibrationsgeber verwendet wird.

Bei symmetrisch und paarweise bezogen auf die Mittellinie des Gürtels angeordneten Vibrationsgebern werden bevorzugt die unmittelbar beidseitig der Mittellinie befindlichen Vibrationsgeber permanent mit der höchstfrequenten Steuerspannung der Teilspektren angesteuert.

Die benannten analogen Bauteile der Elektronik können selbstverständlich auch durch funktionsgleiche digitale Schaltungen und/oder Software realisiert werden.

Nachfolgend werden wesentliche Verfahrensmerkmale benannt, mit denen bei Signalanteilen der Musik in einem nichtfühlbaren Teilspektrum auch diese Signale fühlbar gemacht werden können.

So sieht das Verfahren zur Steigerung der Musikemotionalität, indem aus der hörbaren Musik abgeleitete mit der hörbaren Musik korrelierende Vibrationen von Vibrationsgebern auf die Haut übertragen werden, wobei die Vibrationsgeber räumlich verteilt in einen um die Hüfte zu tragenden Gürtel angeordnet sind und von in unterschiedliche Frequenzbereiche gefilterten Musiksignalen als Steuerspannung angesteuert werden vor, dass
ausgehend von einer mittleren spektralen Breite eines Basisspektrums aus dem Musikspektrum, das Basisspektrum in Teilspektren unterteilt ist, mit den zeitlichen Verläufen der Teilspektren die Vibrationsgeber angesteuert werden und zwischen der Frequenzlage der Steuerspannungen und der Position der Vibrationsgeber ein rechentechnisch abrufbares Zuordnungsschema besteht, wobei bei Signalanteilen der Musik in einem nichtfühlbaren Teilspektrum,
die im höchsten noch fühlbaren Teilspektrum liegende Steuerspannung in der Amplitude angehoben wird, und/oder
ab einer vorgebbaren Amplitude dieser Anteile die im höchsten noch fühlbaren Teilspektrum liegende Steuerspannung mehreren Vibrationsgebern als Steuerspannung zugeführt wird, und/oder
dieser Frequenzbereich über mindestens zwei Filterstufen in seiner Gesamtheit erfasst wird, und/oder
der Selektionsverstärker zur Trennung des nichtfühlbaren vom fühlbaren Frequenzbereich eine dynamische Arbeitspunkteinstellung aufweist, derart, dass das gleichgerichtete Ausgangssignal des Verstärkers gegenphasig über eine zeitliche Verzögerungsstufe auf den Verstärkereingang rückgekoppelt wird, wodurch eine von der Änderungssteilheit des im nichtfühlbaren Frequenzbereich liegenden Musiksignals abhängige Verstärkung erreicht wird, und dass auf diese Weise erzeugte Ausgangssignal des Verstärkers der im fühlbaren Bereich liegenden Oszillatorspannung als Amplitudenverlauf aufgeprägt wird, welche dann als Steuerspannung für die Vibrationsgeber verwendet wird und/oder
bei Signalanteilen der Musik in einem nichtfühlbaren Teilspektrurn und bei einer nur sehr geringen Steuerspannung im höchsten noch fühlbaren Teilspektrum eine Oszillatorspannung, deren Frequenz an der Fühlbarkeitsgrenze liegt, die Funktion der Steuerspannung übernimmt.

Bei Signalanteilen der Musik in einem nicht fühlbaren Teilspektrum sieht das Zuordnungsschema der Steuerspannungen zu den Vibrationsgebern bei mindestens zwei Filterstufen bevorzugt vor dass mit den aus den höherfrequenten Signal abgeleiteten Steuerspannungen die mehr zur Mittellinie des Gürtels hin befindlichen Vibrationsgeber angesteuert werden und mit den aus dem niederfrequenten Signal abgeleiteten Steuerspannungen, die von der Mittelinie weiter entfernten Vibrationsgeber.

Bei einer Amplitudendominanz einer Steuerspannung können weitere Vibrationsgeber mit dieser Spannung angesteuert werden.

Hinsichtlich der Erläuterung dieser Merkmale wird auf die vorangestellten verfahrenstechnischen Ausführungen verwiesen. Diese gelten hier analog.

Die Erfindung wird beispielhaft anhand der Zeichnungen erläutert werden. Es zeigen:
- Fig. 1: eine Vorrichtung mit Vibrationsgebern in Form eines Gürtels und
- Fig. 2: eine Schaltungsanordnung zur Umsetzung eines Eingangssignals.

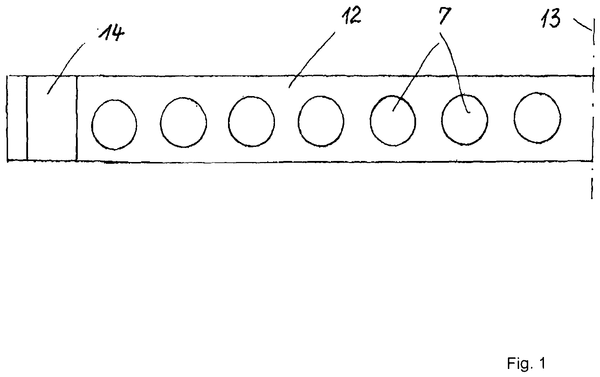

Fig. 1 zeigt die Vorrichtung zur Steigerung der Musikemotionalität in Form eines Gürtels 12, von dem hier nur eine Hälfte und die Innenseite dargestellt sind - angedeutet durch die Mittellinie 13.

In dem Gürtel 12 sind in dem gezeigten Ausführungsbeispiel beidseitig der Mittellinie 13 jeweils sieben Vibrationsgeber angeordnet, vorzugsweise derart, dass sie von Hüftseite zu Hüftseite bei angelegtem Gürtel 12 am Körper anliegen. Es versteht sich, dass auch mehr oder weniger Vibrationsgeber vorgesehen sein können.

Integriert in den Gürtel 12 ist eine Steuerung oder Elektronik 14 mit einer Schnittstelle für eine Musikaufnahmekomponente 1, Die Schnittstelle kann im Falle einer digitalen Steuerung auch direkt ein digitales Eingangssignal empfangen, vorzugsweise drahtlos, z.B. via Bluetooth. Von der Elektronik 14 aus werden die Vibrationsgeber 7 angesteuert. Dies erfolgt paarweise und symmetrisch bezogen auf die Mittellinie 13. Es versteht sich, dass die Vibrationsgeber 7 anstatt paarweise auch einzeln oder in anderen Gruppen von zwei, drei oder mehr Vibrationsgebern angesteuert werden können.

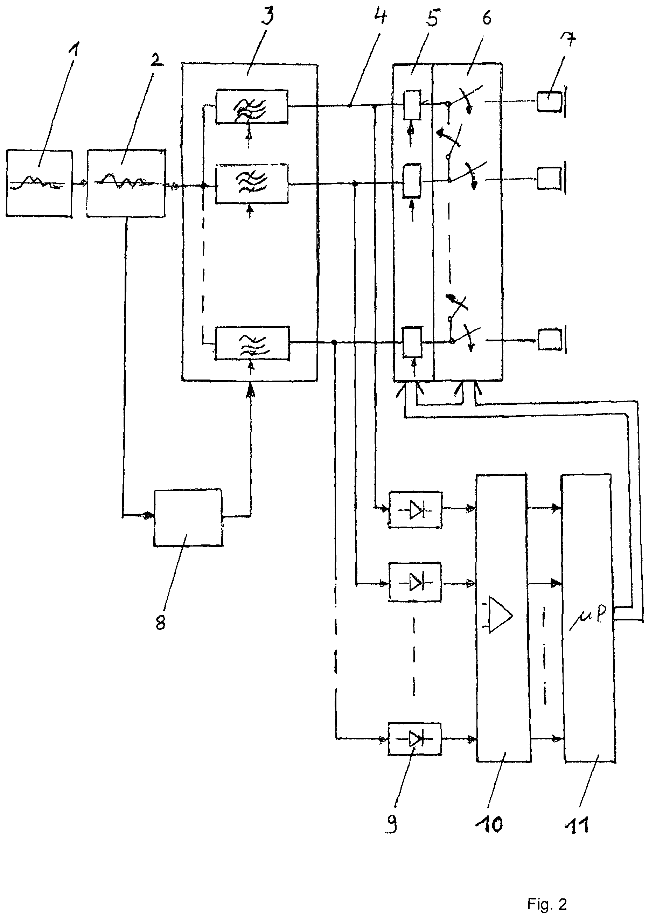

Fig. 2 zeigt die Elektronik 14 zur Ansteuerung der Vibrationsgeber 7 in Form einer Schaltungsanordnung, mit der aus der hörbaren Musik abgeleitete mit der hörbaren Musik korrelierende Vibrationen von Vibrationsgebern 7 auf die Haut übertragen werden. Dabei sind die Vibrationsgeber 7 in einem um die Hüfte bzw. Taille zu tragenden Gürtel 12 angeordnet und werden von Steuerspannungen. die gefilterte und in der Amplitude angepasste Musiksignale sind, angesteuert.

Die Elektronik 14 besteht aus schaltungstechnisch miteinander gekoppelte Komponenten, nämlich eine Musikaufnahmekomponente 1, eine Schaltung zur Ableitung des Basisspektrums 2, steuerbare Fiiterschaltungen 3 mit einer Schaltung 8 zur Anpassung der Filterschaltungen 3, Schaltungen zur Amplitudenerkennung 9, Komparatorschaltungen 10, eine digitale Schaltung 11 zur Umsetzung des Schaltschemas, steuerbare Verstärker 5 sowie eine Schaltmatrix 6 für die Vibrationsgeber 7.

Bei der Musikaufnahmekomponente 1 handelt es sich bevorzugt um eine funktechnisch arbeitende Komponente, d. h. die Musiksignale, die der Träger des Gürtels 12 aus einem Lautsprecher oder per Kopfhörer durch das Sinnenorgan Ohr wahrnimmt, werden zeitgleich durch die Musikaufnahmekomponente 1 aufgenommen und den weiteren Komponenten der Elektronik 14 zur Erzeugung der Steuerspannungen für die Vibrationsgeber 8 zur Verfügung gestellt.

Nach dem Signaleingang in die Musikaufnahmekomponente 1 erfolgt in der Schaltung 2 eine Ableitung des Basisspektrums, wobei ausgehend von einer mittleren spektralen Breite eines Basisspektrums aus dem Musikspektrum, welches sich z. B. von 250 Hz - 8 KHz erstrecken kann, wobei das Basisspektrum in Teilspektren unterteilt ist das Basisspektrum in der Breite erweitert wird unter Beibehaltung der Anzahl der Teilspektren, sobald eine Spektralkomponente der aktuellen Musik auftritt, die außerhalb des aktuellen Basisspektrums liegt oder das Basisspektrum in der Breite reduziert wird unter Beibehaltung der Anzahl der Teilspektren, wenn in einer Zeiteinheit keine Spektralkomponente der aktuellen Musik in den äußeren Bereichen des aktuellen Basisspektrums auftritt.

Mit den gefilterten zeitlichen Verläufen der Teilspektren werden die Vibrationsgeber 7 dann angesteuert, wobei zwischen der Frequenzlage der Steuerspannungen und der Position der Vibrationsgeber 7 ein rechentechnisch abrufbares Zuordnungsschema besteht.

Die steuerbaren Filterschaltungen 3 analysieren nicht jeden Ton, sondern die Teilspektren, in denen die Töne liegen. Den Tönen wird somit eine Frequenz zugeordnet.

Das Basisspektrum umfasst dabei die für die Haut fühlbaren Bereiche z. B. von
55 Hz - 93 Hz
94 Hz - 160 Hz
161 Hz - 280 Hz
281 Hz - 460 Hz und
461 Hz - 820 Hz.
und Musiksignalfrequenzen in nichtfühlbaren Spektren in den Bereichen
821 Hz - 1600 Hz und
1601 Hz - 3200 Hz.

Generell kann davon ausgegangen werden, dass der Übergang zwischen fühl- und nichtfühlbaren Frequenzen durch die Haut bei ca. 1000 Hz liegt.

Parallel zur spektralen Aufteilung der Signale erfolgt durch die Schaltung 8 zur Amplitudenerkennung der gefilterten Musiksignale die Feststellung der Amplituden der Teilspektren bezogen auf ihre Größe und in den anschließenden Komparatorschaltungen ein Vergleich der Größe der Amplituden.

Die folgenden Beispiele fallen nicht in den Bereich der beanspruchten Erfindung. f

In Abhängigkeit von der festgestellten Größe der Amplituden werden in der digitalen Schaltung 11 zur Umsetzung des vorprogrammierten Schaltschemas und damit zum Ansprechen der Vibrationsgeber 7 entsprechende Signale für die Schaltmatrix 6 generiert.

Die Schaltmatrix 6 ist so ausgelegt, dass beliebige Vibrationsgeber 7 und auch Kombinationen von Vibrationsgebern 7 mit den Steuerspannungen aus den Filterschaltung 3 aktiviert werden können

Mit der vorgeschlagenen Verfahrensweise wird kein neues Signal generiert, das mittels der Vibrationsgeber 7 umsetzbar ist, sondern das gefilterte und in der Amplitude angepasste Musiksignal wird direkt übertragen, wobei mittels der Elektronik 14 bestimmt wird, welcher Vibrationsgeber 7 angesteuert wird. Die wesentlichsten Ansteuerungen sind bei den Verfahren ausführlich dargestellt.

Durch die direkte Umsetzung des Musiksignals in Vibrationen, die örtliche Einwirkungsverteilung der gefilterten Musiksignale, die Hervorhebung der dominanten Musiksignale durch Erweiterung der Einwirkungsbreite, die Umsetzung der im nichtfühlbaren Bereich liegenden Signalanteile in den fühlbaren Bereich und das variable, sich dem aktuell vorliegenden Musikspektrum anpassenden Basisspektrum wird eine höchstmögliche Korrelation zwischen gehörten und gefühlten Wahrnehmungen erreicht.

## Patentansprüche

1. Verfahren zur Steigerung der Wahrnehmung von akustischen Ereignissen, insbesondere zur Steigerung der Musikemotionalität, wobei
- aus einem akustischen Ereignis abgeleitete und mit dem akustischen Ereignis korrelierende Vibrationen mittels Vibrationsgebern (7) auf die Haut einer Person übertragen werden,
- die Vibrationsgeber an Positionen in einer vorgegebenen Anordnung auf der Haut räumlich verteilt sind,
- mittels einer Steuerung (14), welcher ein das akustische Ereignis wiedergebende Eingangssignal zugeführt wird, Steuerspannungen bestimmt werden und die Vibrationsgeber jeweils mit einer der ermittelten Steuerspannungen zur Erzeugung einer auf der Haut fühlbaren Vibration angesteuert werden,
- aus dem Frequenzspektrum des akustischen Ereignisses ein Basisspektrum (2) bestimmt und das Basisspektrum in Teilspektren unterteilt wird, welche jeweils einen Frequenzbereich des Basisspektrums umfassen,
- die Steuerspannungen jeweils aus dem Frequenzbereich zumindest eines zugeordneten Teilspektrums abgeleitet werden,
- zwischen Frequenzlagen der Steuerspannungen und den Positionen der Vibrationsgeber und / oder zwischen den Teilspektren und den Positionen der Vibrationsgeber eine Zuordnung besteht,
- der Amplitudenverlauf des im nicht fühlbaren Frequenzbereich liegenden Eingangssignals einer im fühlbaren Frequenzbereich liegenden Oszillationsspannung als Amplitudenverlauf aufgeprägt und diese als Steuerspannung den Vibrationsgebern zugeführt wird, und
- ein Selektionsverstärker (5) den nicht fühlbaren vom fühlbaren Frequenzbereich trennt und eine dynamische Arbeitspunkteinstellung aufweist, derart,
- dass ein gleichgerichtetes Ausgangssignal des Verstärkers (5) gegenphasig über eine zeitliche Verzögerungsstufe auf den Verstärkereingang rückgekoppelt wird, wodurch eine von der Änderungssteilheit des im nicht fühlbaren Frequenzbereich liegenden Eingangssignals abhängige Verstärkung erreicht wird, und
- dass der Amplitudenverlauf des auf diese Weise erzeugten Ausgangssignals des Verstärkers einer im fühlbaren Bereich liegenden Oszillatorspannung als Amplitudenverlauf aufgeprägt wird, welche dann als Steuerspannung für die Vibrationsgeber verwendet wird.

2. Verfahren nach Anspruch 1, wobei
- das Bestimmen des Basisspektrums ein Erweitern und / oder Reduzieren des Basisspektrums in der Breite unter Beibehaltung der Anzahl der Teilspektren umfasst,
- das Basisspektrum in der Breite reduziert wird, wenn in einer Zeiteinheit eine Anzahl von Spektralkomponenten im Frequenzbereich des akustischen Ereignisses in den Randbereichen des aktuellen Basisspektrums auftritt, die unterhalb eines vorgegebenen Schwellenwerts liegt, insbesondere keine Spektralkomponente, und
- das Basisspektrum in der Breite erweitert wird, wenn eine Spektralkomponente im Frequenzspektrum des akustischen Ereignisses auftritt, die außerhalb des ausgewählten Basisspektrums liegt.

3. Verfahren nach Anspruch 1 oder 2, wobei
- die Anzahl der von einer Steuerspannung angesteuerten Vibrationsgeber vom Amplitudenverhältnis der einzelnen Steuerspannungen abhängig ist,
- bei im Wesentlichen gleicher Amplitude der Steuerspannungen aller Teilspektren die Steuerspannungen den Vibrationsgebern in der Reihenfolge ihrer Frequenzlage zugeführt werden und / oder
- ab einer vorgegebenen Abweichung zwischen den Amplituden der Steuerspannungen die dominierende Steuerspannung dem Vibrationsgeber des zugeordneten Teilspektrums sowie einem oder mehreren Vibrationsgebern von Teilspektren mit einer höheren Frequenzlage zugeführt werden.

4. Verfahren nach einem der vorhergehenden Ansprüche, wobei mindestens einer der Vibrationsgeber permanent mit der höchstfrequenten Steuerspannung angesteuert wird.

5. Verfahren nach einem der vorhergehenden Ansprüche, wobei mit der Steuerspannung, welche in der Frequenzlage unterhalb der Steuerspannung mit dem maximalen Wert die höchste Amplitude aufweist, auch ein oder mehrere Vibrationsgeber, welche zwischen diesen beiden Steuerspannungen liegen, angesteuert werden und eine sinngemäße Zuordnung für die Steuerspannungen in den darunter liegenden Frequenzbereichen besteht.

6. Verfahren nach einem der vorhergehenden Ansprüche, wobei
- die Vibrationsgeber in einer sich entlang einer Verteilungsrichtung erstreckenden Anordnung auf der Haut verteilt angeordnet sind,
- die Vibrationsgeber vorzugsweise symmetrisch und paarweise bezogen auf eine Mittellinie (13) der Anordnung angeordnet sind und paarweise oder einzeln angesteuert werden,
- sich weiter vorzugsweise die Frequenz der Steuerspannungen in Richtung der Mittellinie erhöht, und
- vorzugsweise zumindest die unmittelbar beidseitig der Mittellinie befindlichen Vibrationsgeber permanent mit der höchstfrequenten Steuerspannung angesteuert werden.

7. Verfahren nach einem der vorhergehenden Ansprüche, wobei
- bei Auftreten von Signalanteilen des Eingangssignals in einem nicht fühlbaren Teilspektrum die im höchsten noch fühlbaren Teilspektrum liegende Steuerspannung in der Amplitude angehoben wird, und / oder
- bei Auftreten von Signalanteilen des Eingangssignals in einem nicht fühlbaren Teilspektrum ab einer vorgegebenen Amplitude dieser Signalanteile die im höchsten noch fühlbaren Teilspektrum liegende Steuerspannung mehreren Vibrationsgebern als Steuerspannung zugeführt wird.

8. Verfahren nach einem der vorhergehenden Ansprüche, wobei
- bei Signalanteilen des Eingangssignals in einem nicht fühlbaren Teilspektrum der Frequenzbereich dieses Teilspektrums über eine oder mehrere, vorzugsweise mindestens zwei Filterstufen in seiner Gesamtheit erfasst wird,
- vorzugsweise bei Signalanteilen des Eingangssignals in einem nicht fühlbaren Teilspektrum die Zuordnung der Steuerspannungen zu den Vibrationsgebern bei einer oder mehreren, vorzugsweise mindestens zwei Filterstufen derart vorgenommen wird, dass mit den aus einem höherfrequenten Signalanteil abgeleiteten Steuerspannungen näher an der Mittellinie angeordnete Vibrationsgeber angesteuert werden und mit den aus einem niederfrequenten Signalanteil abgeleiteten Steuerspannungen von der Mittelinie weiter entfernte Vibrationsgeber angesteuert werden, und
- weiter vorzugsweise bei einer Amplitudendominanz einer Steuerspannung zusätzlich zu dem zugeordneten Vibrationsgeber zumindest ein weiterer Vibrationsgeber, insbesondere ein zu dem zugeordneten Vibrationsgeber benachbarter Vibrationsgeber mit dieser Steuerspannung angesteuert wird.

9. Verfahren nach einem der vorhergehenden Ansprüche, wobei bei akustischen Ereignissen mit eingeschränktem Spektralbereich die Aufteilung der Teilspektren für die Steuerspannungen entsprechend der Gesamtbreite des Frequenzspektrums gespreizt wird.

10. Verfahren nach einem der vorhergehenden Ansprüche, wobei über eine Benutzeroberfläche, vorzugsweise über eine Software, Steuerbefehle zur Veränderung einer Programmierung und / oder der Zuordnung übermittelbar sind.

11. Vorrichtung zur Steigerung der Wahrnehmung akustischer Ereignisse, welche eingerichtet ist, das Verfahren nach einem der vorhergehenden Ansprüche auszuführen, die Vorrichtung aufweisend:
- eine Mehrzahl von Vibrationsgebern zum Übertragen von Vibrationen auf die Haut einer Person, wobei die Vibrationsgeber an Positionen in einer vorgegebenen Anordnung derart angeordnet sind, dass sie auf der Haut der Person räumlich verteilbar sind,
- eine mit den Vibrationsgebern verbundene Steuerung, die für das Verfahren nach einem der vorhergehenden Ansprüche und hierbei für Folgendes eingerichtet ist:
- die Vibrationsgeber mit einer Steuerspannung zur Erzeugung der Vibrationen anzusteuern und
- ein zu dem akustischen Ereignis gehörendes Eingangssignal zu empfangen, Steuerspannungen zu bestimmen und die Vibrationsgeber jeweils mit einer der ermittelten Steuerspannungen zur Erzeugung einer auf der Haut fühlbaren Vibration anzusteuern, und
- einen Selektionsverstärker, der eingerichtet ist, den nicht fühlbaren vom fühlbaren Frequenzbereich zu trennen, und eine dynamische Arbeitspunkteinstellung aufweist, derart eingerichtet, dass
- ein gleichgerichtetes Ausgangssignal des Verstärkers gegenphasig über eine zeitliche Verzögerungsstufe auf den Verstärkereingang rückzukoppeln, wodurch eine von der Änderungssteilheit des im nicht fühlbaren Frequenzbereich liegenden Eingangssignals abhängige Verstärkung erreicht wird, und
- den Amplitudenverlauf des auf diese Weise erzeugten Ausgangssignals des Verstärkers einer im fühlbaren Bereich liegenden Oszillatorspannung als Amplitudenverlauf aufzuprägen, welche dann als Steuerspannung für die Vibrationsgeber verwendet wird,
wobei der Amplitudenverlauf des im nicht fühlbaren Frequenzbereich liegenden Eingangssignals einer im fühlbaren Frequenzbereich liegenden Oszillationsspannung als Amplitudenverlauf aufprägbar und diese als Steuerspannung den Vibrationsgebern zuführbar ist,
wobei die Vorrichtung weiter eingerichtet ist, aus einem akustischen Ereignis abgeleitete und mit dem akustischen Ereignis korrelierende Vibrationen mittels den Vibrationsgebern auf die Haut einer Person zu übertragen.

12. Vorrichtung nach Anspruch 11, wobei die Vibrationsgeber (7) in einer sich entlang einer Verteilungsrichtung erstreckenden Anordnung angeordnet sind, vorzugsweise symmetrisch und paarweise bezogen auf eine Mittellinie (13) der Anordnung, und paarweise oder einzeln ansteuerbar sind.

13. Vorrichtung nach Anspruch 11 oder 12, wobei die Vorrichtung als ein um die Hüfte zu tragender Gürtel, als Band oder als Kleidungsstück ausgebildet ist, vorzugsweise derart, dass bei Benutzung die Vibrationsgeber auf der Haut im Bereich des Bauchs und der Hüfte der Person platzierbar sind.

14. Vorrichtung nach einem der Ansprüche 11 bis 13, wobei die Vibrationsgeber (7) derart angeordnet sind, dass sie beim Tragen der Vorrichtung durch die Person mit der Haut der Person in Kontakt kommen oder derart relativ zur Haut der Person angeordnet sind, dass eine Vibration auf die Haut übertragbar ist.

15. Vorrichtung nach einem der Ansprüche 11 bis 14, wobei die Steuerung zum Empfang des Eingangssignals mindestens eine Schnittstelle, insbesondere eine drahtlose Schnittstelle, und / oder eine Musikaufnahmeeinrichtung (1) zur Aufnahme von Schallwellen des akustischen Ereignisses aufweist.

## Claims

1. Method for increasing the perception of acoustic events, in particular, for increasing musical emotionality, wherein
- vibrations derived from an acoustic event and correlated with the acoustic event are transmitted to the skin of a person by means of vibration transmitters (7),
- the vibration transmitters are spatially distributed at positions in a specified arrangement on the skin,
- by means of controller (14), to which an input signal reproducing the acoustic event is supplied, control voltages are determined, and the vibration transmitters are each controlled using one of the determined control voltages to generate a vibration that is perceptible on the skin,
- a basic spectrum (2) is determined from the frequency spectrum of the acoustic event and the basic spectrum is divided into sub-spectra, each comprising a frequency range of the basic spectrum,
- the control voltages are derived from the frequency range of at least one assigned sub-spectrum,
- there is an assignment between the frequency layers of the control voltages and the positions of the vibration transmitters and/or between the sub-spectra and the positions of the vibration transmitters,
- the amplitude course of the input signal in the non-perceptible frequency range of an oscillation voltage in the perceptible frequency range is superimposed as an amplitude course and this is supplied to the vibration transmitters as control voltage, and
- a selection amplifier (5) separates the non-perceptible from the perceptible frequency range and comprises a dynamic operating point setting in such a way
- that a rectified output signal of the amplifier (5) is fed back in phase opposition via a temporal delay stage to the amplifier input, whereby a gain, which depends on the variability of the input signal in the non-perceptible frequency range, is achieved, and
- that the amplitude course of the output signal of the amplifier generated in this way is superimposed, as an amplitude course, to an oscillator voltage in the perceptible range, which is then used as control voltage for the vibration generators.

2. The method according to Claim 1, wherein
- the determination of the basic spectrum includes an expansion and/or reduction of the basic spectrum in width while maintaining the number of sub-spectra,
- the basic spectrum is reduced in width if a number of spectral components in the frequency range of the acoustic event occurs in a unit of time in the peripheral areas of the current basic spectrum that is below a specified threshold value, in particular no spectral component, and
- the basic spectrum is extended in width when a spectral component occurs in the frequency spectrum of the acoustic event that lies outside the selected basic spectrum.

3. The method according to Claim 1 or 2, wherein
- the number of vibration transmitters controlled by a control voltage depends on the amplitude ratio of the individual control voltages,
- at essentially the same amplitude of the control voltages of all sub-spectra, the control voltages are supplied to the vibration transmitters in the order of their frequency position and/or
- from a specified deviation between the amplitudes of the control voltages, the dominant control voltage is supplied to the vibration transmitter of the assigned sub-spectrum and to one or a plurality of vibration transmitters of sub-spectra at a higher frequency position.

4. The method according to any one of the preceding claims, wherein at least one of the vibration transmitters is permanently controlled using the high-frequency control voltage.

5. The method according to any one of the preceding claims, wherein, using the control voltage that has the highest amplitude at the frequency position below the control voltage with the maximum value, one or a plurality of vibration transmitters that lie between these two control voltages are also controlled and there is a corresponding assignment for the control voltages in the underlying frequency ranges.

6. The method according to any one of the preceding claims, wherein
- the vibration transmitters are distributed on the skin in an arrangement extending along a direction of distribution,
- the vibration transmitters are preferably symmetrically and arranged in pairs in relation to a centre line (13) of the arrangement and are controlled in pairs or individually,
- further preferably, the frequency of the control voltages increases in the direction of the centre line, and
- preferably, at least the vibration transmitters located directly on both sides of the centre line are continuously controlled using the highest frequency control voltage.

7. The method according to any one of the preceding claims, wherein,
- in the event of the occurrence of signal components of the input signal in a non-perceptible sub-spectrum, the amplitude of the control voltage within the highest sub-spectrum that is still perceptible is raised, and/or,
- if signal components of the input signal occur in a non-perceptible sub-spectrum from a specified amplitude of these signal components, the control voltage within the highest sub-spectrum that is still perceptible is supplied to a plurality of vibration transmitters as control voltage.

8. The method according to any one of the preceding claims, wherein,
- in the case of signal components of the input signal in a non-perceptible sub-spectrum, the frequency range of this sub-spectrum is detected in its entirety via one or a plurality of filter stages, preferably at least two of them,
- preferably, in the case of signal components of the input signal in a non-perceptible sub-spectrum, the assignment of the control voltages to the vibration transmitters at one or a plurality of filter stages, preferably, at least two of them is carried out in such a way that, vibration transmitters arranged closer to the centre line are controlled using the control voltages derived from a higher frequency signal component and vibration transmitters distanced further away from the centre line are controlled using the control voltages derived from a low-frequency signal component, and
- more preferably, at an amplitude dominance of a control voltage in addition to the assigned vibration transmitter at least one further vibration transmitter, in particular a vibration transmitter adjacent to the assigned vibration transmitter is controlled using this control voltage.

9. The method according to any one of the preceding claims, wherein in the case of acoustic events with a limited spectral range, the division of the sub-spectra for the control voltages is spread according to the total width of the frequency spectrum.

10. The method according to any one of the preceding claims, wherein via a user interface, preferably via a software, control commands for changing a programming and/or the assignment are transmittable.

11. Device for increasing the perception of acoustic events, which is set up to carry out the method according to any one of the preceding claims, the device comprising:
- a plurality of vibration transmitters for transmitting vibrations to the skin of a person, wherein the vibration transmitters are arranged at positions in a specified arrangement in such a way that they are spatially distributable on the skin of the person,
- a controller connected to the vibration transmitters, which is set up for the method according to any one of the preceding claims and herein for the following:
- to control the vibration transmitters using a control voltage to generate the vibrations, and
- to receive an input signal belonging to the acoustic event, to determine control voltages and to control the vibration transmitters using one of the determined control voltages to generate a vibration that can be felt on the skin, and
- a selection amplifier, which is designed to separate the non-perceptible from the perceptible frequency range and comprises a dynamic operating point setting, configured
- to feed back a rectified output signal of the amplifier in phase opposition via a temporal delay stage to the amplifier input, whereby a gain dependent on the partial change of the input signal in the non-perceptible frequency range is achieved, and
- to superimpose the amplitude course of the output signal of the amplifier generated in this way of an oscillator voltage in the perceptible range as an amplitude course, which is then used as control voltage for the vibration transmitters,
wherein the amplitude course of the input signal in the non-perceptible frequency range of an oscillation voltage in the perceptible frequency range can be superimposed as an amplitude course and this can be supplied to the vibration transmitters as control voltage,
wherein the device is further set up to transmit vibrations derived from an acoustic event and correlated with the acoustic event to the skin of a person by means of the vibration transmitters.

12. The device according to Claim 11, wherein the vibration transmitters (7) are arranged in an arrangement extending along a distribution direction, preferably symmetrical and pairwise related to a centre line (13) of the arrangement, and pairwise or individually controllable.

13. The device according to Claim 11 or 12, wherein the device is formed as a belt to be worn around the hip, as a band or as a garment, preferably in such a way that, when used, the vibration transmitters can be placed on the skin in the area of the abdomen and hip of the person.

14. The device according to any one of the Claims 11 to 13, wherein the vibration transmitters (7) are arranged in such a way that they come into contact with the skin of the person when the device is worn by the person or are arranged so relative to the skin of the person that a vibration is transmissible to the skin.

15. The device according to any one of the Claims 11 to 14, wherein the controller for receiving the input signal comprises at least one interface, in particular a wireless interface, and/or a music recording device (1) for recording sound waves of the acoustic event.

## Revendications

1. Procédé, destiné à accroître la perception d'évènements acoustiques, en particulier destiné à accroître l'émotivité musicale, lors duquel
- des vibrations dérivées d'un évènement acoustique et en corrélation avec l'événement acoustique sont transmises à l'aide de générateurs de vibrations (7) sur la peau d'une personne,
- les générateurs de vibrations sont physiquement répartis sur des positions selon une configuration prédéfinie sur la peau,
- à l'aide d'un système de commande (14), auquel est amené un signal d'entrée restituant l'événement acoustique, des tensions de commande sont déterminées et les générateurs de vibrations sont activés chaque fois avec l'une des tensions de commande identifiées, pour générer une vibration perceptible sur la peau,
- à partir du spectre de fréquences de l'événement actuel, un spectre de base (2) est déterminé, et le spectre de base est divisé en spectres partiels, lesquels comprennent chacun une gamme de fréquences du spectre de base,
- les tensions de commande sont dérivées chaque fois de la gamme de fréquences d'au moins un spectre partiel associé,
- il persiste une association entre des positions de fréquences des tensions de commande et les positions des générateurs de vibrations et/ ou entre les spectres partiels et les positions des générateurs de vibrations,
- la courbe d'amplitude du signal d'entrée se situant dans la gamme de fréquences non perceptible d'une tension d'oscillation qui se situe dans la gamme de fréquences perceptible est appliquée en tant que courbe d'amplitude et celle-ci est amenée en tant que tension de commande aux générateurs de vibrations, et
- un amplificateur de sélection (5) sépare la gamme de fréquences non perceptible de la perceptible et comporte un ajustement dynamique du point de fonctionnement, de telle sorte
- qu'un signal de sortie redressé de l'amplificateur (5) soit rétro-couplé en opposition de phase, via un étage de retard temporel sur l'entrée de l'amplificateur, suite à quoi l'on obtient une amplification dépendant de la pente de variation du signal d'entrée se situant dans la gamme de fréquences non perceptibles, et
- que la courbe d'amplitude du signal de sortie de l'amplificateur généré de cette manière soit appliquée à une tension d'oscillateur se situant dans la gamme perceptible en tant que courbe d'amplitude, qui est alors utilisée en tant que tension de commande pour les générateurs de vibrations.

2. Procédé selon la revendication 1, lors duquel
- la détermination du spectre de base comprend un élargissement et / ou une réduction du spectre de base dans la largeur, en maintenant le nombre des spectres partiels,
- le spectre de base est réduit dans la largeur, si dans une unité de temps, il se produit dans les zones marginales du spectre de base actuel un nombre de composantes spectrales dans la gamme de fréquences de l'évènement acoustique qui est inférieur à une valeur seuil prédéfinie, en particulier aucune composante spectrale, et
- le spectre de base est élargi dans la largeur, s'il se produit une composante spectrale dans le spectre de fréquences de l'évènement acoustique, qui se situe hors du spectre de base sélectionné.

3. Procédé selon la revendication 1 ou 2, lors duquel
- le nombre des générateurs de vibrations activés par une tension de commande dépend d'un rapport d'amplitude entre les tensions de commande individuelles,
- dans le cas d'une amplitude sensiblement égale des tensions de commande de tous les spectres partiels, les tensions de commande sont amenées aux générateurs de vibrations selon la séquence de leur position de fréquence et / ou
- à partir d'un écart prédéfini entre les amplitudes des tensions de commande, la tension de commande dominante est amenée au générateur de vibrations du spectre partiel associé, ainsi qu'à un ou à plusieurs générateurs de vibrations de spectres partiels assortis d'une position de fréquences plus élevée.

4. Procédé selon l'une quelconque des revendications précédentes, lors duquel au moins l'un des générateurs de vibrations est activé en permanence avec la tension de commande assortie de la fréquence la plus élevée.

5. Procédé selon l'une quelconque des revendications précédentes, lors duquel, à l'aide de la tension de commande qui dans la position de fréquences en-dessous de la tension de commande assortie de la valeur maximale comporte l'amplitude la plus élevée, également un ou plusieurs générateurs de vibrations, lesquels se situent entre lesdites deux tensions de commande sont activés et une association subsiste pour les tensions de commande relevant des gammes de fréquences situées en-dessous.

6. Procédé selon l'une quelconque des revendications précédentes, lors duquel
- les générateurs de vibrations sont placés sur la peau selon une configuration s'étendant le long d'une direction de répartition,
- les générateurs de vibrations sont placés de préférence de manière symétrique et par paires en rapport à une ligne médiane (13) de la configuration et sont activés par paires ou individuellement,
- de manière préférentielle par ailleurs, la fréquence des tensions de commande augmente en direction de la ligne médiane, et
- de préférence, au moins les générateurs de vibrations se trouvant directement de part et d'autre de la ligne médiane sont activés en permanence avec la tension de commande assortie de la fréquence la plus élevée.

7. Procédé selon l'une quelconque des revendications précédentes, lors duquel
- lors de la production de fractions de signal du signal d'entrée dans un spectre partiel non perceptible, la tension de commande qui se situe dans le spectre partiel le plus élevé encore perceptible est relevée en amplitude, et/ ou
- lors de la production de fractions de signal du signal d'entrée dans un spectre partiel non perceptible, à partir d'une amplitude prédéfinie desdites fractions de signal, la tension de commande qui se situe dans le spectre partiel le plus élevé encore perceptible est amenée en tant que tension de commande vers plusieurs générateurs de vibrations.

8. Procédé selon l'une quelconque des revendications précédentes, lors duquel
- dans le cas de fractions de signal du signal d'entrée dans un spectre partiel non perceptible, la gamme de fréquences dudit spectre partiel est détectée dans sa totalité via un ou plusieurs, de préférence au moins deux étages de filtrage,
- de préférence, dans le cas de fractions de signal du signal d'entrée dans un spectre partiel non perceptible, il est procédé à l'association des tensions de commande aux générateurs de vibrations sur un ou plusieurs, de préférence au moins deux étages de filtrage, de telle sorte qu'avec les tensions de commande dérivées d'une fraction de signal assortie d'une fréquence plus élevée, des générateurs de vibrations placés plus près de la ligne médiane soient activés et qu'avec les tensions de commande dérivées d'une fraction de commande assortie d'une plus faible fréquence, des générateurs de vibrations plus éloignés de la ligne médiane soient activés, et
- de manière préférentielle par ailleurs, dans le cas d'une dominance d'amplitude d'une tension de commande, en supplément du générateur de vibrations associé, au moins un autre générateur de vibrations, en particulier un générateur de vibrations voisin du générateur de vibrations associé est activé à l'aide de ladite tension de commande.

9. Procédé selon l'une quelconque des revendications précédentes, lors duquel dans le cas d'évènements acoustiques à domaine spectral restreint, la répartition des spectres partiels pour les tensions de commande est étalée en fonction de la largeur partielle du spectre de fréquences.

10. Procédé selon l'une quelconque des revendications précédentes, lors duquel, par l'intermédiaire d'une interface utilisateur, de préférence d'un logiciel, des ordres de commande destinés à modifier une programmation et / ou à procéder à l'association sont transmissibles.

11. Dispositif, destiné à accroître la perception d'évènements acoustiques, lequel est aménagé pour réaliser le procédé selon l'une quelconque des revendications précédentes, le dispositif comportant :
- une multiplicité de générateurs de vibrations, destinés à transmettre des vibrations sur la peau d'une personne, les générateurs de vibrations étant placés à des positions selon une configuration prédéfinie de telle sorte qu'ils puissent être répartis physiquement sur la peau de la personne,
- un système de commande, connecté avec les générateurs de vibrations, qui est aménagé pour le procédé selon l'une quelconque des revendications précédentes et à cet effet, pour ce qui suit :
- pour activer les générateurs de vibrations avec une tension de commande, pour générer les vibrations, et
- pour réceptionner un signal d'entrée qui fait partie de l'évènement acoustique, pour déterminer des tensions de commande et pour activer les générateurs de vibrations chaque fois avec l'une des tensions de commande identifiées, pour générer une vibration perceptible sur la peau, et
- un amplificateur de sélection, qui est aménagé pour séparer les gammes de fréquences non perceptibles des perceptibles et qui comporte un ajustement dynamique du point de fonctionnement, aménagé de sorte
- à rétro-coupler en opposition de phase sur l'entrée de l'amplificateur, via un étage de retard temporel un signal de sortie redressé l'amplificateur, ce qui permet d'obtenir une amplification dépendant de la pente de variation du signal d'entrée se situant dans la gamme de fréquences non perceptible, et
- à appliquer la courbe d'amplitude du signal de sortie de l'amplificateur généré de cette manière à une tension d'oscillateur se situant dans la gamme perceptible en tant que courbe d'amplitude, qui est alors utilisée en tant que tension de commande pour les générateurs de vibrations,
la courbe d'amplitude du signal d'entrée qui se situe dans la gamme de fréquences non perceptible étant susceptible d'être appliquée à une tension d'oscillateur se situant dans la gamme de fréquences perceptible en tant que courbe d'amplitude et celle-ci pouvant être amenée en tant que tension de commande aux générateurs de vibrations,
le dispositif étant aménagé par ailleurs, pour transmettre sur la peau d'une personne, à l'aide des générateurs de vibration des vibrations dérivées d'un évènement acoustique et en corrélation avec l'évènement acoustique.

12. Dispositif selon la revendication 11, les générateurs de vibrations (7) étant placés selon une configuration qui s'étend le long d'une direction de répartition, de préférence de manière symétrique et par paires, en rapport à une ligne médiane (13) de la configuration, et étant susceptibles d'être activés par paires ou individuellement.

13. Dispositif selon la revendication 11 ou 12, le dispositif étant conçu sous la forme d'une ceinture à porter autour des hanches, d'une bande ou d'un vêtement, de préférence de telle sorte que lors de l'utilisation, les générateurs de vibrations puissent être placés sur la peau, dans la région du ventre et des hanches de la personne.

14. Dispositif selon l'une quelconque des revendications 11 à 13, les générateurs de vibrations (7) étant placés de telle sorte que lorsque le dispositif est porté par la personne, ils entrent en contact avec la peau de la personne, ou soient placés par rapport à la peau de la personne de sorte qu'une vibration puisse être transmise sur la peau.

15. Dispositif selon l'une quelconque des revendications 11 à 14, pour réceptionner le signal d'entrée, le système de commande comportant au moins une interface, en particulier une interface sans fil, et / ou un système d'enregistrement musical (1), destiné à enregistrer des ondes sonores de l'évènement acoustique.
